# EUROPEAN PATENT SPECIFICATION

(11) **EP 4 504 974 B1**
(45) Date of publication and mention of the grant of the patent: **11.11.2026**
(21) Application number: 23719007.9
(22) Date of filing: 06.04.2023
(51) Int. Cl.: C12Q 1/6883

(54) **IMPROVED DIAGNOSIS OF NONALCOHOLIC STEATOHEPATITIS**
VERBESSERTE DIAGNOSE VON NICHT-ALKOHOLISCHER STEATOHEPATITIS
DIAGNOSTIC AMÉLIORÉ DE LA STÉATOHÉPATITE NON ALCOOLIQUE

(30) Priority: 08.04.2022 EP 22305503
(43) Date of publication of application: 12.02.2025
(73) Proprietor: Genfit, 59120 Loos (FR)
(72) Inventor: MAGNANENSI, Jérémy, 59800 LILLE (FR); ROSENQUIST, Christian, 92200 Neuilly-sur-Seine (FR); MAJD, Zouher, 59320 ENNETIERES-EN-WEPPES (FR); HAJJI, Yacine, 59000 LILLE (FR)
(74) Representative: Cabinet Becker et Associés
(86) International application number: PCT/EP2023/059290
(87) International publication number: WO 2023/194593

(56) References cited:
- WO-A1-2017/167934
- WO-A1-2022/029066
- HARRISON STEPHEN A ET AL: "A blood-based biomarker panel (NIS4) for non-invasive diagnosis of non-alcoholic steatohepatitis and liver fibrosis: a prospective derivation and global validation study", vol. 5, no. 11, 1 November 2020 (2020-11-01), US, pages 970 - 985, XP055792366, ISSN: 2468-1253, Retrieved from the Internet <URL:http://dx.doi.org/10.1016/S2468-1253(20)30252-1> DOI: 10.1016/S2468-1253(20)30252-1
- HYDES T ET AL: "Current and Emerging Biomarkers and Imaging Modalities for Nonalcoholic Fatty Liver Disease: Clinical and Research Applications", CLINICAL THERAPEUTICS, ELSEVIER, AMSTERDAM, NL, vol. 43, no. 9, 13 August 2021 (2021-08-13), pages 1505 - 1522, XP086852353, ISSN: 0149-2918, [retrieved on 20210813], DOI: 10.1016/J.CLINTHERA.2021.07.012
- HARRISON STEPHEN ET AL: "Baseline levels of NIS4(TM) and other fibrosis biomarkers and prediction of histological progression to advanced fibrosis in nash", vol. 72, 1 November 2020 (2020-11-01), pages 896a - 897a, XP055955200, Retrieved from the Internet <URL:https://aasldpubs.onlinelibrary.wiley.com/doi/epdf/10.1002/hep.31579>
- ANSTEE QUENTIN ET AL: "IDENTIFICATION OF PATIENTS WITH AT-RISK NASH OR ADVANCED FIBROSIS USING NIS4(TM) ALONE OR IN COMBINATION AS COMPARED WITH OTHER TESTING STRATEGIES", vol. 72, no. 1, 1 October 2020 (2020-10-01), pages 920a - 921a, XP055955205, Retrieved from the Internet <URL:https://aasldpubs.onlinelibrary.wiley.com/doi/10.1002/hep.31579>
- VALERIA ANNARITA PIAZZOLLA ET AL: "Noninvasive Diagnosis of NAFLD and NASH", CELLS, vol. 9, no. 4, 17 April 2020 (2020-04-17), pages 1005, XP055758520, DOI: 10.3390/cells9041005

## Description

### BACKGROUND OF THE INVENTION

Nonalcoholic steatohepatitis (NASH) is a chronic liver disease characterized histologically by the accumulation of fat, hepatocyte damage and inflammation resembling alcoholic hepatitis. NASH can lead to liver fibrosis, cirrhosis, liver failure and/or hepatocellular carcinoma (HCC). Until recently, NASH was largely underdiagnosed, as it carries no obvious symptoms in its early stages, and also because of the lack of widely available non-invasive tests specifically developed to diagnose the disease. At-risk NASH status, which is defined as having NASH, NAS score 4 and fibrosis stage F2, represents an important NASH sub-population to identify. Indeed, these patients are associated to elevated risks of disease worsening, notably cirrhosis, and higher risk of liver-related and all-cause mortality. Due to the technical limitations and also risks of biopsies, which are the clinical reference standard for the diagnosis of NASH and fibrosis, the development of blood-based non-invasive tests (NITs) is of major importance. Different NITs have been developed, mainly to fit with fibrosis stages. NIS4^{®} is a first-in-class blood-based NIT that is specifically designed for addressing a composite Fibrosis x NAS endpoint by detecting at-risk NASH patients, and contains 4 independent biomarkers: miR-34a-5p, alpha-2 macroglobulin (A2M), YKL-40 (or chitinase 3-like protein 1) and glycated hemoglobin (HbA1c) (Harrison et al., The Lancet Gastroenterology & Hepatology, 5(11):970-985, 2020). NIS4^{®} allows performing robust analysis. NIS4^{®} is based on the evaluation of 4 different biomarkers requiring different biological fluids (whole blood and serum), and different lab processes. In this context, we evaluated whether it would be possible to provide a NIT improved over the NITs of the prior art.

### SUMMARY OF THE INVENTION

The present invention is based on the detailed analysis of significant datasets of clinical trials. It is herein provided an optimized non-invasive method for the identification of at-risk NASH subjects.

Accordingly, disclosed herein is a method for the diagnosis of at-risk NASH in a subject, wherein said method comprises quantifying the levels of miR-34a-5p and YKL-40 in a biological fluid sample of said subject and combining the quantified levels with the gender of the subject.

More specifically, the invention relates to a method for the diagnosis, screening, and monitoring of at-risk NASH in a subject, said method comprising:
- quantifying the levels of miR-34a-5p and YKL-40 in a biological fluid sample of said subject;
- obtaining the gender of said subject;
- combining the quantified levels and the gender in a mathematical function to assign a score; and
- comparing said score with cutoff values to determine whether said subject is of risk of having an at-risk NASH,
wherein the score is calculated according to the formula disclosed on page 8.

In a particular embodiment, the method of the invention is for the diagnosis of at-risk NASH in a subject.

In a further particular embodiment, the score is compared with cutoff values to determine whether said subject is of high, low or indeterminate risk of having an at-risk NASH.

In a particular embodiment, the mathematical function includes a logistic regression function. In another embodiment, the biological fluid sample is a blood, serum or plasma sample, preferably a serum sample.

In yet another embodiment, the subject suffers from obesity, insulin resistance, glucose intolerance, type 2 diabetes mellitus (T2DM), prediabetes, dyslipidaemia or hypertriglyceridaemia.

According to another aspect, the invention relates to a computer program comprising instructions that, when executed by a processor/processing means, cause the processor/processing means to:
- receive quantified levels of miR34a-5p and YKL-40;
- receive the gender of the subject;
- calculate a score from these quantified levels and gender of the subject, from a mathematical function as described herein; and
- assign the subject into the group of at-risk subjects or not at-risk subjects based upon the calculated score compared to predetermined cutoff values.

In yet another aspect, the invention relates to a computer readable medium comprising the computer program disclosed herein. In a particular embodiment, the computer readable medium is a non-transitory medium or a storage medium.

In addition, the present invention relates to specific anti-NASH or anti-fibrotic agents for use in the treatment of at-risk NASH in a subject in need thereof, wherein the subject has been classified as having at-risk NASH thanks to the method disclosed herein.

### DETAILED DESCRIPTION OF THE INVENTION

The present invention relates to a non-invasive method than can be used to aid discrimination between at-risk NASH and not at-risk NASH in a subject, from a biological fluid sample of the subject.

Histological scoring/staging systems have been developed to assess NAFLD activity level and fibrosis stage and to estimate the risk of evolution to clinical liver outcomes. The NALFD-Activity-Score (NAS) has been developed to assess the severity of NAFLD. NAS is the sum of three histological scores determined from liver biopsy slices:
- S: Steatosis score: 0: <5%; 1: 5-33%; 2: 34-66% and 3: >66%;
- LI: Lobular Inflammation score (foci per 20x field): 0: none; 1: <2 foci; 2: 2-4 foci and 3: >4 foci; and
- HB: Ballooning degeneration score: 0: none; 1: few; 2: many cells/prominent ballooning.

Using this scoring system, a "patient with NASH" has NAS≥3, with at least 1 point in steatosis, at least 1 point in lobular inflammation and at least 1 point in hepatocyte ballooning. A "non-NASH" patient is a patient having either (i) a NAS≥3 with at least one of steatosis, lobular inflammation and hepatocyte ballooning scores equal to 0; or (ii) a NAS<3. In addition, in the context of the present invention, a patient is excluded as being a NASH patient if said patient has viral hepatitis, autoimmune liver disease, alcohol-related liver disease, drug-induced liver disease or congenital causes of chronic liver disease such as hereditary hemochromatosis, Wilson's disease, alpha-1-antitrypsin deficiency and polycystic ovary syndrome.

Localization and extent of fibrosis (F) at histological exam signs the severity (advancement) of NASH. The NASH-CRN (Nonalcoholic SteatoHepatitis Clinical Research Network) has developed a dedicated fibrosis staging system (Kleiner, D.E et al, Hepatology, 2005 Jun; 41(6):1313-21).

| **NASH Clinical Research Network Scoring System Definitions** | **F Score** |
|---|---|
| Perisinusoidal or periportal fibrosis | 1 |
| Mild perisinusoidal fibrosis (zone 3) | 1a |
| Moderate perisinusoidal fibrosis (zone 3) | 1b |
| Portal/periportal fibrosis | 1c |
| Perisinusoidal and portal/periportal fibrosis | 2 |
| Bridging fibrosis | 3 |
| Cirrhosis | 4 |

Using this fibrosis staging system, patients with no or minimal fibrosis (F=0-1) are generally not considered at risk of cirrhosis, liver failure, HCC (hepatocellular carcinoma) or liver-related death. Patients with significant (F=2) and advanced fibrosis (F=3) are at increasing risk of developing cirrhosis, liver failure, HCC and liver-related death. Patients with compensated cirrhosis have severe fibrosis (F=4) and are at high risk of liver failure (decompensated cirrhosis), HCC and liver-related deaths. Identifying patients who are at risk of developing HCC, cirrhotic complications and liver-related deaths is the ultimate reason for liver assessment. As defined by the FDA and EMA, patients at risk of liver outcomes who should be pharmacologically treated are those with NAS≥4 (with score ≥ 1 for each of steatosis, lobular inflammation and ballooning) and NASH-CRN fibrosis score (F) ≥ 2.

Accordingly, in the context of the present invention, a patient with "at-risk NASH", otherwise referred to as an "at-risk patient" or as a "patient at risk of hepatic outcome", is a patient with a NAS higher or equal to 4, a S score higher or equal to 1, a LI score higher or equal to 1, a HB score higher or equal to 1 and a F score of higher or equal to 2. It defines a subgroup of NASH patients having a high risk of developing at least one life-threatening liver outcome such as cirrhosis, liver failure, HCC and liver-related death.

The terms "subject" and "patient" may be used interchangeably herein and refer to a human subject. As mentioned above, NASH occurs more commonly in patients suffering from metabolic disorders. In addition, NASH is known to be associated to comorbidities such as metabolic disorders. Therefore, the method of the present invention can more particularly benefit to those patients presenting such comorbidities. Common comorbidities of NASH include obesity, insulin resistance, glucose intolerance, T2DM, prediabetes, dyslipidaemia, hypertriglyceridaemia, hypertension and cardiovascular disease. Older age may also predispose to HCC in NASH patients. Therefore, in a particular embodiment, the patient suffers from a metabolic disorder, such as obesity, insulin resistance, glucose intolerance, T2DM, prediabetes, dyslipidaemia and hypertriglyceridaemia.

As used herein, the expression "biological fluid sample" refers to a blood, serum or plasma sample, preferably a serum sample.

In a particular embodiment, as used herein the expression "screening" refers to the selection of a patient to be treated or not to be treated in a cohort or in a clinical study. The screening of patients may imply a diagnostic step and then the distribution of the patient in a group: to be treated and not to be treated.

In the present invention, the selection of at-risk NASH patients among NASH patients depends on the score calculated with the levels of hsa-miR34a, YKL40, and the sex of the patient. The further comparison of the score with the cutoff value can be used to assign the patient in a group of patients who will receive a treatment or in a group of patients who will not receive a treatment.

In the context of the present invention, a patient to be treated is a patient at-risk of NASH and a patient not to be treated is a patient not at-risk of NASH.

As used herein, the term "monitoring" refers to a comparison of the score calculated at two time points. Monitoring is the ongoing, systematic collection and analysis of data as a project or condition progresses, like a clinical study or a treatment protocol. According to the invention, the score increases during time, the pathology progresses whereas if the score decreases the pathology regresses. The monitoring is possible due to the noninvasiveness of the method according to the present invention. Indeed, the ease of use of this assay allows repetitive measures and the follow-up of a patient in the time course of a pathology. In conclusion, the monitoring is just the application of the method of diagnosis along time for a patient.

Analysis of a biological fluid sample can be performed using several analytical methods, depending on the type of biomarkers to be quantified. Such analytical methods include quantitative RT-PCR, mass spectrometry, immunoPCR and immunodetection. One can also cite the use of a biochip to implement the simultaneous analysis of multiple biomarkers.

In a particular embodiment, the level of miR-34a-5p is measured as Cq (amplification cycle) or fold change, preferably as fold. Suitable methods are readily available to those skilled in the art. Specific RNAs can be quantified after extraction of total RNA from the biological fluid sample, using commercial kits for example. Extraction efficiency can be monitored to minimize sample-to-sample variation. Such monitoring can be conducted by addition of an internal process control (IPC) to the sample before total RNA extraction. Such IPC can correspond to a miRNA molecule of known sequence which is heterologous to the sample. For example, one can use a miRNA molecule found in a different species than human, such as a miRNA molecule from *Caenorhabditis elegans,* in particular Cel-miR-40-3p (SEQ ID NO:1: UCACCGGGUGUACAUCAGCUAA). Such IPC can be contained in synthetic vesicles. To quantify the level of miR-34a-5p, positive controls with a known miR-34a-5p Cq value can be used, such as three positive controls with a known miR-34a-5p Cq value. These positive controls can cover the range of miR-34a-5p level in NASH population. In case three positive controls are used, one corresponds to a low miR-34a-5p level, one to a medium level and another to a high level. The medium level can also be referred to as the calibrator, serving in the assay to calculate the fold change value. The positive controls are processed at the same time as the tested sample of the subject. Reverse transcription is carried out simultaneously on i) extracted total RNA from samples spiked with IPC and ii) on total RNA from positive controls spiked with the IPC as well, for example using a commercial reverse transcription kit. Quantification can then be implemented on cDNA using, for example, a commercial quantitative PCR kit. Cq determination mode can be regression. Moreover, for each patient sample, transcript abundance can be expressed as fold change using both IPC and calibrators Cqs according to equations used to normalize the calibrator against the IPC, to normalize the sample against the IPC, and to quantify miR-34a-5p level as fold change.

In the following paragraph, we describe a specific, but non-limiting method to achieve miR34a-5p quantification. Briefly, total RNA is extracted from a patient serum samples, using Promega magnetic bead-based extraction Maxwell^{®} Plasma and Serum Kit (AS1680, Promega) and RCS48 Instrument (AS8500, Promega) according to the manufacturer's instructions. To monitor extraction efficiency and to minimise sample-to-sample variation, synthetic vesicles containing *Caenorhabditis elegans* Cel-miR-40-3p (Mature miRNA sequence UCACCGGGUGUACAUCAGCUAA-3', Integrated DNA Technologies, purification RNAse free HPLC) are used as IPC and are added to each sample prior to RNA extraction. Three positive controls with a known miR-34a Cq values (low [C1=32 Cq], medium also called calibrator [C2=30.7 Cq], and high [C3=28Cq] has-miR34a-5p levels) covering the range of miR-34a expression in NASH population are processed at the same time of tested samples with the medium standard serving also as calibrator to the assay to calculate the fold change value. Total RNA from serum samples, containing IPC and total RNA from positive controls containing IPC as well are concomitantly reverse transcribed using TaqMan MicroRNA Reverse Transcription Kit (4366597, Applied Biosystems, Thermo Fisher Scientific). Reverse transcription reaction is carried out in a final mixture of 24 µL containing 3µL of TaqMan MicroRNA Assay 5X and incubated in a Thermal Cycler T100 (Biorad). cDNAs are stored in low binding tubes at -20°C until further use. Expression of mature miRNAs is quantified according to the manufacturer's instructions using the TaqMan miRNA RT-qPCR Assay 20X and TaqMan Universal Master Mix II, no Uracil-N-Glycosilase (UNG) (4440040, Applied Biosystems, ThermoFisher Scientific). A fixed volume of 5 µL of total cDNA is used as a template for the qPCR assay using a CFX96 Real-Time PCR detection System. The miR-34a TaqMan assay (sequence of mature hsa-miR-34a-5p = UGGCAGUGUCUUAGCUGGUUGU (SEQ ID NO:2), miR-base number=MIMAT0000255; and sequence of mature Cel-miR-40-3p= UCACCGGGUGUACAUCAGCUAA (SEQ ID NO:1), miR-base number= MIMAT0000011) is used. The Cq determination mode is regression. For each patient sample, miRNA level is expressed as fold change using both IPC and calibrators Cqs according to the following equations:
- Step 1: Normalization of calibrator C2 against Internal Process Control $Δ {Cq}_{C 2 miR - 34} = {Cq}_{C 2 miR - 34 a} - {Cq}_{C 2 miR - 40 in C 2}$
- Step 2: Normalization of sample against Internal Process Control $Δ {Cq}_{Sample miR - 34 a} = {Cq}_{sample miR - 34 a} - {Cq}_{sample miR - 40}$
- Step 3: Sample miR-34a delta expression calculation in Cq $ΔΔ {Cq}_{sample miR - 34} = Δ {Cq}_{Sample} - Δ {Cq}_{C 2}$
- Step 4: Sample delta expression of miR-34a expressed as fold $hsa - miR - 34 a Fold Change \left(FC\right) = {2}^{- ΔΔCq}$

In a particular embodiment, the level of YKL-40 is measured in ng/ml. Methods to determine the level of a protein such as YKL-40 in a biological fluid sample are readily available to those skilled in the art. For example, once can cite methods based on immunodetection, such as ELISA.

The method further comprises determining the gender of the subject. It is herein shown that gender has a major influence on the output of a score implementing both miR-34a-5p and YKL-40 levels. Surprisingly, the level of these biomarkers and the gender of the subject can be used to develop a mathematical function (i.e. a statistical algorithm) which can accurately predict the probability of at-risk NASH.

Thus, preferably, each of the biomarker level and the gender of the subject can be introduced into a mathematical function to produce an output value that correlates with at-risk NASH status. The method thus can be used to discriminate subjects as having at-risk NASH or not having at-risk NASH. One skilled in the art is aware of numerous suitable methods for developing mathematical function, and all of these are within the scope of the present invention. In a particular embodiment, the mathematical function includes a logistic regression equation.

In a further embodiment, the method of the present invention implements the following formula: $S = \frac{1}{1 + exp \left(-y\right)}$ wherein 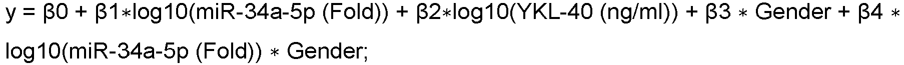 y = β0 + β1*log10(miR-34a-5p (Fold)) + β2*log10(YKL-40 (ng/ml)) + β3 * Gender + β4 * log10(miR-34a-5p (Fold)) * Gender; and wherein Gender is 0 if the subject is a female, or 1 if the subject is a male.

In a particular embodiment, β0 is comprised between -3 and 3, in particular between -2 and 2. In a particular embodiment, β1 is comprised between 1 and 5, in particular between 2 and 4. In a particular embodiment, β2 is comprised between 0 and 4.5, in particular between 0.5 and 3. In a particular embodiment, β3 is comprised between -2 and 2, in particular between -1 and 1. In a particular embodiment, β4 is comprised between -1 and 2, in particular between 0 and 2. In a further particular embodiment, β0 is comprised between -3 and 3, β1 is comprised between 1 and 5, β2 is comprised between 0 and 4.5, β3 is comprised between -2 and 2 and β4 is comprised between -1 and 2. In yet another particular embodiment, β0 is comprised between -2 and 2, β1 is comprised between 2 and 4, β2 is comprised between 0.5 and 3, β3 is comprised between -1 and 1 and β4 is comprised between 0 and 2.

By way of example, the following equations can be used for the diagnosis of at-risk NASH: equation 1: 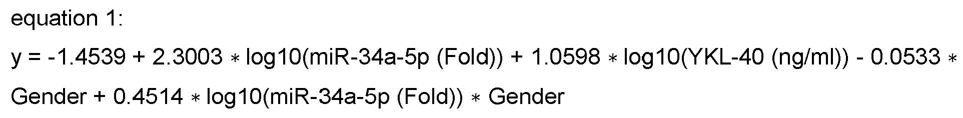 y = -1.4539 + 2.3003 * log10(miR-34a-5p (Fold)) + 1.0598 * log10(YKL-40 (ng/ml)) - 0.0533 * Gender + 0.4514 * log10(miR-34a-5p (Fold)) * Gender equation 2: 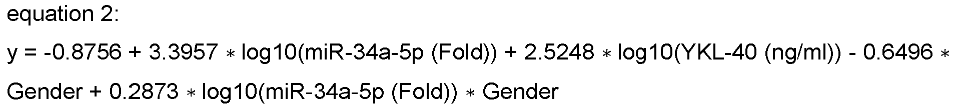 y = -0.8756 + 3.3957 * log10(miR-34a-5p (Fold)) + 2.5248 * log10(YKL-40 (ng/ml)) - 0.6496 * Gender + 0.2873 * log10(miR-34a-5p (Fold)) * Gender equation 3: 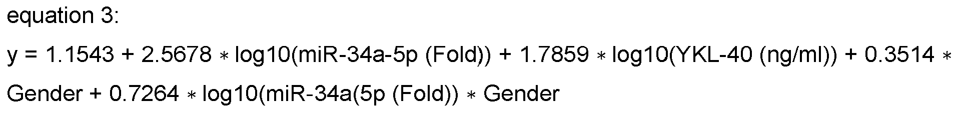 y = 1.1543 + 2.5678 * log10(miR-34a-5p (Fold)) + 1.7859 * log10(YKL-40 (ng/ml)) + 0.3514 * Gender + 0.7264 * log10(miR-34a(5p (Fold)) * Gender

The score calculated from the mathematical function can then be compared to predetermined cutoff values, such as low and high cutoff values. In this context, a calculated S value lower that the low cutoff is indicative of a subject not having at-risk NASH, and a calculated S value greater or equal to the high cutoff value is indicative of a subject having at-risk NASH.

In a particular embodiment, the low cutoff is comprised between 0.24 and 0.5, in particular between 0.41 and 0.49. In another particular embodiment, the high cutoff is comprised between 0.6 and 0.95, in particular between 0.62 and 0.74. In a further particular embodiment, the low cutoff is equal to 0.4564. In a further particular embodiment, the high cutoff is equal to 0.6815. In a further particular embodiment, the low cutoff is equal to 0.4564 and the high cutoff is equal to 0.6815.

The present invention also relates to a computer program comprising instructions that, when executed by a processor/processing means, cause the processor/processing means to:
- receive quantified levels of miR-34a-5p and YKL-40;
- receive the gender of the subject;
- calculate a score from these quantified levels and gender of the subject, from a mathematical function as described above; and
- assign the subject into the group of at-risk subjects or not at-risk subjects based upon the calculated score compared to predetermined cutoff values.

The present invention further provides a computer readable medium comprising the computer program described therein. According to a particular embodiment, the computer readable medium is non-transitory medium or a storage medium.

In some embodiments, thanks to the method of the invention, a decision may be taken to give life-style recommendations to a subject (such as a food regimen or providing physical activity recommendations), to medically take care of a subject (e.g. by setting regular visits to a physician or regular examinations, for example for regularly monitoring markers of liver damage), or to administer at least one NASH or liver fibrosis therapy to the patient, to treat or prevent at-risk NASH. In a particular embodiment, a decision may be taken to give life-style recommendations to a subject or to administer at least one NASH or liver fibrosis therapy. The invention thus further relates to an anti-NASH or anti-fibrotic compound for use in a method for treating NASH in a subject in need thereof, wherein the subject has been identified thanks to a method according to the invention.

The term "treatment", as used herein, relates to both therapeutic measures and prophylactic or preventive measures, wherein the goal is to prevent or slow down (lessen) an undesired physiological change or disorder. Beneficial or desired clinical results include, but are not limited to, alleviation of symptoms, stabilizing pathological state (specifically not worsening), slowing down or stopping the progression of the disease, improving or mitigating the pathological condition. Particularly, for the purpose of the present invention, treatment is directed to slow the progression of NASH and/or fibrosis and reduce the risk of further complications. It can also involve prolonging survival in comparison with the expected survival if the treatment is not received.

The anti-NASH or anti-fibrotic agent is administered in a therapeutically effective amount. As used herein, the expression "therapeutically effective amount" refers to an amount of the drug effective to achieve a desired therapeutic result. A therapeutically effective amount of a drug may vary according to factors such as the disease state, age, sex, and weight of the individual, and the ability of drug to elicit a desired response in the individual. A therapeutically effective amount is also one in which any toxic or detrimental effects of agent are outweighed by the therapeutically beneficial effects. The effective dosages and dosage regimens for drug depend on the disease or condition to be treated and may be determined by the persons skilled in the art. A physician having ordinary skill in the art may readily determine and prescribe the effective amount of the pharmaceutical composition required. For example, the physician could start doses of drug employed in the pharmaceutical composition at levels lower than that required in order to achieve the desired therapeutic effect and gradually increase the dosage until the desired effect is achieved. In general, a suitable dose of a composition of the present invention will be that amount of the compound which is the lowest dose effective to produce a therapeutic effect according to a particular dosage regimen. Such an effective dose will generally depend upon the factors described above.

In a particular embodiment, the invention relates to an anti-NASH compound for use in a method for treating NASH in a subject suffering from at-risk NASH, wherein the subject has been classified as having at-risk NASH thanks to the method according to the invention.

Illustrative anti-NASH and anti-fibrotic compounds are listed below:
- a compound of formula (I) or a pharmaceutically acceptable salt thereof: 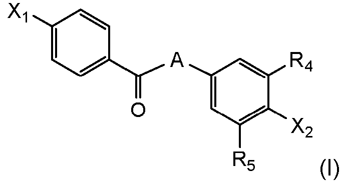 wnerein:
   X1 represents a halogen atom, a R1 group or G1-R1 group;
   A represents a CH=CH or CH2-CH2 group;
   X2 represents a G2-R2 group;
   G1 represents an atom of oxygen;
   G2 represents an atom of oxygen or sulfur;
   R1 represents a hydrogen atom, an unsubstituted alkyl group, an aryl group or an alkyl group that is substituted by one or more substituents selected from halogen atoms, alkoxy groups, alkylthio groups, cycloalkyl groups, cycloalkylthio groups and heterocyclic groups;
   R2 represents an alkyl group substituted by a -COOR3 group, wherein R3 represents a hydrogen atom or an alkyl group that is substituted or not by one or more substituents selected from halogen atoms, cycloalkyl groups and heterocyclic groups; and
   R4 and R5, identical or different, represent an alkyl group that is substituted or not by one or more substituent selected from halogen atoms, cycloalkyl groups and heterocyclic groups;
- AMP activated protein kinase stimulators such as PXL-770, MB-11055, Debio-0930B, metformin, CNX-012, O-304, mangiferin calcium salt, eltrombopag, carotuximab, and imeglimin;
- Bile acids such as obeticholic acid (OCA), ursodeoxycholic acid (UDCA), norursodeoxycholic acid, and ursodiol;
- CCR antagonists such as cenicriviroc (CCR2/5 antagonist), PG-092, RAP-310, INCB-10820, RAP-103, PF-04634817, and CCX-872;
- Dipeptidyl peptidase IV (DPP4) inhibitors such as evogliptin, vidagliptin, fotagliptin, alogliptin, saxagliptin, tilogliptin, anagliptin, sitagliptin, retagliptin, melogliptin, gosogliptin, trelagliptin, teneligliptin, dutogliptin, linagliptin, gemigliptin, yogliptin, betagliptin, imigliptin, omarigliptin, vidagliptin, and denagliptin;
- Farnesoid X receptor (FXR) agonists such as obeticholic acid (OCA), tropifexor (LJN452), cilofexor (GS9674), Nidufexor (LMB763), EDP-305, AKN-083, INT-767, GNF-5120, LY2562175, INV-33, NTX-023-1, EP-024297, Px-103, SR-45023, TERN-101 (6-{4-[5-Cyclopropyl-3-(2,6-dichloro-phenyl)-isoxazol-4-ylmethoxy]-piperidin-1-yl}-1-methyl-1H-indole-3 carboxylic acid), TERN-201, TERN-501 and TERN-301;
- Fibroblast Growth Factor 19 (FGF-19) receptor ligand or functional engineered variant of FGF-19;
- Fibroblast Growth Factor 21 (FGF-21) agonists such as PEG-FGF21 (pegbelfermin, formely BMS-986036), YH-25348, BMS-986171, YH-25723, LY-3025876, and NNC-0194-0499;
- engineered Fibroblast Growth Factor 19 (FGF-19) analogues such as NGM-282 (aldafermin);
- Glucagon-like peptide-1 (GLP-1) analogs such as semaglutide, liraglutide, exenatide, albiglutide, dulaglutide, lixisenatide, loxenatide, efpeglenatide, taspoglutide, MKC-253, DLP-205, and ORMD-0901;
- Nicotinic acid such as Niacin and Vitamin B3;
- nitazoxanide (NTZ), its active metabolite tizoxanide (TZ) or other prodrugs of TZ such as RM-5061;
- PPAR alpha agonists such as fenofibrate, ciprofibrate, pemafibrate, gemfibrozil, clofibrate, binifibrate, clinofibrate, clofibric acid, nicofibrate, pirifibrate, plafibride, ronifibrate, theofibrate, tocofibrate, and SR10171;
- PPAR gamma agonists such as pioglitazone, deuterated pioglitazone, rosiglitazone, efatutazone, ATx08-001, OMS-405, CHS-131, THR-0921, SER-150-DN, KDT-501, GED-0507-34-Levo, CLC-3001, and ALL-4;
- PPAR delta agonists such as GW501516 (Endurabol or ({4-[({4-methyl-2-[4-(trifluoromethyl)phenyl]-1,3-thiazol-5-yl}methyl)sulfanyl]-2-methylphenoxy}acetic acid), MBX8025 (Seladelpar or {2-methyl-4-[5-methyl-2-(4-trifluoromethyl- phenyl)-2H-[I,2,3]triazol-4-ylmethylsylfanyl]-phenoxy}-acetic acid), GW0742 ([4-[[[2-[3-fluoro-4-(trifluoromethyl)phenyl]-4-methyl-5-thiazolyl]methyl]thio]-2-methyl phenoxy]acetic acid), L165041, HPP-593, and NCP-1046;
- PPAR alpha/gamma dual agonists (also named glitazars) such as saroglitazar, aleglitazar, muraglitazar, tesaglitazar, and DSP-8658;
- PPAR gamma/delta dual agonists such as conjugated linoleic acid (CLA), and T3D-959;
- PPAR alpha/gamma/delta pan agonists or PPARpan agonists such as IVA337, TTA (tetradecylthioacetic acid), bavachinin, GW4148, GW9135, bezafibrate, lanifibranor, lobeglitazone, and CS038;
- Sodium-glucose transport (SGLT) 2 inhibitors such as licoglifozin, remogliflozin, dapagliflozin, empagliflozin, ertugliflozin, sotagliflozin, ipragliflozin, tianagliflozin, canagliflozin, tofogliflozin, janagliflozin, bexagliflozin, luseogliflozin, sergliflozin, HEC-44616, AST-1935, and PLD-101.
- stearoyl CoA desaturase-1 inhibitors/fatty acid bile acid conjugates such as aramchol, GRC-9332, steamchol, TSN-2998, GSK-1940029, and XEN-801;
- thyroid receptor β (THR β) agonists such as VK-2809, resmetirom (MGL-3196), MGL-3745, SKL-14763, sobetirome, BCT-304, ZYT-1, MB-07811 and eprotirome;
- Vitamin E and isoforms; vitamin E combined with vitamin C and atorvastatin.

In a particular embodiment, the anti-NASH agent is selected from pegbelfermin, cenicriviroc, dapagliflozin, dulaglutide, empagliflozin, fenofibrate, lanifibranor, liraglutide, obeticholic acid, pioglitazone, resmetirom, saroglitazar magnesium, seladelpar, semaglutide, sitagliptin, TERN-101, TERN-201 and tropifexor.

### EXAMPLES

NIS4^{®} is a first-in-class blood-based NIT that was specifically designed for addressing a composite Fibrosis x NAS endpoint by detecting at-risk NASH patients, and contains 4 independent biomarkers: miR-34a-5p, alpha-2 macroglobulin (A2M), YKL-40 (or chitinase 3-like protein 1) and glycated hemoglobin (HbA1c) (Harrison et al., The Lancet Gastroenterology & Hepatology, 5(11):970-985, 2020). However, NIS4^{®} is based on the evaluation of 4 different biomarkers requiring different biological fluids (whole blood and serum), and different lab processes.

The purpose was thus to improve that NIT while, if possible, achieving as high performance as NIS4^{®} for the detection of at-risk NASH subjects, and to improve the robustness of the model by limiting external factors potentially affecting the NIT performance. The purpose was also to offer a test at reduced costs.

A dataset issued from the Golden-505 clinical trial (NCT01694849) was used as a training cohort. Golden dataset only contains NASH patients, with at least a NAS score equals to 3. Also, no cirrhotic patients are present in this dataset. The final extracted training dataset contains 198 NASH patients, with a prevalence of 50% of at-risk NASH. Both non-at-risk and at-risk NASH populations have been as expected well-balanced for potential confounders by keeping all at-risk NASH patients originally present in the dataset and selecting for each of them an optimal control.

From the Golden dataset, the bayesian information criterion (BIC) for different models including different combinations of biomarkers constituting NIS4^{®} has been calculated. Furthermore, different parameters which can potentially affect the robustness of the test were evaluated, such as age, gender and T2DM status of the patients. These models were all trained using logistic regression. Then, using a dataset derived from the Resolve-It clinical trial (NCT02704403), we determined the robustness of the simplified model. This dataset contains 684 patients, mostly NASH ones (95.32%), with a prevalence of at-risk NASH of 66%. Using this dataset, we built different matched sub-populations to analyze the robustness of the scores obtained with the simplified model.

We used the matchit function, from the Matchlt package, version 4.3.2, with genetic method. In each case, we launched 20 executions in parallel, making the caliper ranging from 0.005 to 0.1 by 0.005. Between all caliper tested, the choice of the caliper was done in order to achieve less than 0.1 in standardized differences for all variables, 0.05 if possible, by also keeping the highest number of patients. By using this process, we obtained in each case sub-populations that are well-balanced for all factors so that the only characteristic that differs between both populations is the one studied. We could thus reasonably assume that this specific factor is the source of impact on the biomarkers, if there is an effect.

Interestingly, the combination of miR-34 and YKL-40 made the model being stable regarding age and T2DM status. On the contrary, gender was found to have an impact on the output of the model. A new modelization was thus conducted to take into account the impact of gender.

In order to try to correct the gender impact in the combination of miR-34a-5p and YKL-40, we decided to train a new model that also contains a "miR-34a-5p x Gender" interaction parameter, with the Golden training cohort described above.

The following formula was derived from this study: $S = \frac{1}{1 + exp \left(-y\right)}$ wherein 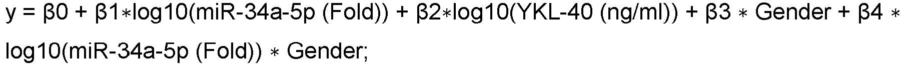 y = β0 + β1*log10(miR-34a-5p (Fold)) + β2*log10(YKL-40 (ng/ml)) + β3 * Gender + β4 * log10(miR-34a-5p (Fold)) * Gender; and wherein Gender is 0 if the subject is a female, or 1 if the subject is a male.

This model is referred to hereinbelow as "GBM".

An example is shown in table 1.

**Table 1:**

| | miR34-a | YKL-40 | Gender | miR34a-5p x Gender |
|---|---|---|---|---|
| miR-34a-5p + YKL-40 | 2.3003 | 1.0598 | -0.0533 | 0.4514 |

Cutoffs were then computed. A low (Lc, 80% sensitivity) cutoff equal to 0.4564 and a high (Hc, 90% specificity) cutoff value equal to 0.6815 were thus calculated.

The performance of this model was then compared to that of NIS4^{®} using patients from the Resolve-it clinical trial. This dataset, referred to as "RIt2", is derived from information obtained from 2035 patients. This represents an independent validation dataset with respect to the Golden dataset used as a training dataset.

As expected, at-risk NASH patients were also associated to higher prevalence of T2DM, Dyslipidemia, hypertension and also obese patients compared to non-at-risk NASH population. Regarding the histological spectrum, we observed an important panel of patients with F3/F4 fibrosis score, but with a NAS score of 3.

We then carried out an AUROC analysis. To start this validation process, we first focused on the overall performances of different tests in detecting at-risk NASH patients. We first started by plotting ROC curves of NIS4^{®}/GBM models, as well as Fibrosis-4 index (FIB-4) and alanine aminotransferase (ALT) ones. Indeed, FIB-4 is often used as reference/surrogate marker for Fibrosis, while ALT could be seen as a surrogate marker for NASH. We then compared the different GBM, FIB-4 and ALT AUROCs to NIS4^{®} one using Delong tests, and results are reported in table 2.

**Table 2: AUROC comparisons vs NIS4^{®} - at-risk NASH endpoint**

| | N | N at-risk | AUROC (95% Cl) | P value |
|---|---|---|---|---|
| NIS4^{®} | 2035 | 929 | 0.7919 (0.7722, 0.8107) | NA |
| GBM | 2035 | 929 | **0.8133** (0.7951, 0.8318) | 2e-04 |
| FIB-4 | 2035 | 929 | 0.6528 (0.6287, 0.6763) | <0.0001 |
| ALT | 2035 | 929 | 0.6986 (0.6766, 0.721) | <0.0001 |

GBM globally outperformed the other NITs, including NIS4^{®} , for the detection of at-risk NASH patients returning an AUROC of 0.81.

GBM overall performances have been compared on different endpoints than at-risk NASH, following the NIMBLE process. For that, we decided to analyze 3 different endpoints mainly focusing on NASH (at-risk NASH F3, NASH, NAS4), and 3 fibrotic only endpoints (F2, F3, F4). For the first ones, we included ALT in comparators as reference, and for the second group, FIB-4 was included as reference. Results are respectively summarized in Tables 3 and 4.

**Table 3: AUROC comparison vs NIS4 - different NASH related endpoints**

| At-risk F3 (26.1%) | | NASH (66.0%) | | NAS4 (64.5%) | | |
|---|---|---|---|---|---|---|
| AUROC (95% Cl) | P value | AUROC (95% Cl) | P value | AUROC (95% Cl) | P value | |
| NIS4^{®} | 0.8 (0.79, 0.83) | NA | 0.74 (0.71, 0.76) | NA | 0.72 (0.7, 0.75) | NA |
| GBM | 0.79 (0.77, 0.81) | 0.0113 | 0.78 (0.76, 0.8) | <0.0001 | 0.78 (0.76, 0.8) | <0.0001 |
| ALT | 0.66 (0.64, 0.69) | <0.0001 | 0.7 (0.68, 0.72) | 0.0054 | 0.73 (0.71, 0.76) | 0.3689 |

**Table 4: AUROC comparison vs NIS4 - different fibrotic endpoints**

| | F2 (59.1%) | | F3 (35.2%) | | F4 (6.5%) | |
|---|---|---|---|---|---|---|
| | AUROC (95% Cl) | P value | AUROC (95% Cl) | P value | AUROC (95% Cl) | P value |
| NIS4^{®} | 0.82 (0.8, 0.84) | NA | 0.79 (0.77, 0.81) | NA | 0.77 (0.74, 0.81) | NA |
| GBM | 0.81 (0.79, 0.83) | 0.037 | 0.74 (0.72, 0.77) | <0.0001 | 0.69 (0.65, 0.73) | <0.0001 |
| FIB-4 | 0.72 (0.69, 0.74) | <0.0001 | 0.74 (0.72, 0.77) | 1e-04 | 0.78 (0.75, 0.82) | 0.6882 |

GBM kept high performances across the different NASH-oriented endpoints, improving also the performances of NIS4^{®} on the NAS4 endpoint.

We then focused on the comparison of clinical performances of NIS4^{®} vs GBM for ruling-out and ruling-in patients for at-risk NASH with their respective Lc and Hc determined above. Results are summarized in Table 5.

**Table 5: NIS4^{®} vs GBM clinical performances comparison**

| | NIS4^{®} | GBM | P value |
|---|---|---|---|
| AUC | 0.7919 (0.7722, 0.8107) | 0.8133 (0.7951, 0.8318) | 2e-04 |

| **Rule-out** | | | |
|---|---|---|---|
| Lc | 0.3614 | 0.4564 | 0.2695 |
| SenLc | 86.11 (83.68, 88.24) | 85.04 (82.54, 87.24) | <0.0001 |
| SpeLc | 53.07 (50.08, 56.04) | 61.21 (58.26, 64.08) | 0.3437 |
| NPV | 81.98 (78.93, 84.69) | 82.97 (80.17, 85.45) | 1e-04 |
| **Indeterminate** | 566 (27.81) | 474 (23.29) | |

| **Rule-in** | | | |
|---|---|---|---|
| Hc | 0.6282 | 0.6815 | |
| SenHe | 58.77 (55.52, 61.95) | 62.11 (58.89, 65.23) | 0.0134 |
| SpeHc | 81.28 (78.83, 83.52) | 84.81 (82.53, 86.85) | 8e-04 |
| PPV | 72.51 (69.14, 75.64) | 77.45 (74.24, 80.37) | 1e-04 |

Regarding the ruling-out performances, for Lc that make both NITs to achieve similar Sensitivity (85-86%) and NPV (82-83%), we observed significant increase in specificity for GBM vs NIS4^{®}.

Concerning the ruling-in performances, GBM achieved significant higher values of specificity (85% vs 81%), PPV (77% vs 73%) as compared to NIS4^{®}. GBM also returned significant higher sensitivity compared to NIS4^{®} (62% vs 59%).

Finally, when focusing on the Indeterminate Zone, again results were in favor of GBM.

Subpopulation analyses were also performed and compared between NIS4^{®} and GBM. See Table 6 below.

**Table 6: inter-group means comparisons of NIS4^{®} and GBM**

| | | | | NIS4^{®} | | GBM | |
|---|---|---|---|---|---|---|---|
| | | | Prev | means ± sd | P value | means ± sd | P value |
| **Gender (n=1318)** | | | | | | | |
| | Female | | 52.2 | 0.509 ± 0.26 | NA | 0.564 ± 0.27 | NA |
| | Male | | 52.2 | 0.541 ± 0.26 | 0.0235 | 0.564 ± 0.27 | 0.9755 |

| **Age (n=738)** | | | | | | | |
|---|---|---|---|---|---|---|---|
| | | ≤50 | 46.1 | 0.463 ± 0.27 | NA | 0.54 ± 0.28 | NA |
| | | ≥60 | 46.1 | 0.557 ± 0.25 | <0.0001 | 0.554 ± 0.26 | 0.4921 |

| **T2DM (n=1284)** | | | | | | | |
|---|---|---|---|---|---|---|---|
| | | N | 47.7 | 0.493 ± 0.25 | NA | 0.556 ± 0.27 | NA |
| | | Y | 47.7 | 0.577 ± 0.24 | <0.0001 | 0.562 ± 0.26 | 0.7051 |

While NIS4^{®} was impacted by age and T2DM status, GBM scores were no longer significantly impacted by age and T2DM status. We thus obtained a GBM modelization that was robust on these factors.

GBM also efficiently reduced the impact of gender on the scores. We observed that the correction on GBM scores was mainly performed on low values, corresponding to the observation that the gender effect on miR-34a-5p was mainly present on not at-risk NASH patients.

Therefore, we have identified a simplified and more robust NIT as compared to NIS4^{®}. This new model is based on a reduced number of biomarkers, the quantification of both of them being performed in serum.

Interestingly, this new model requires only one biological fluid sample to perform the markers analysis. For this reason, this NIT is also cheaper and easier to implement. Moreover, we have demonstrated that this new NIT is less impacted by gender, age and T2DM status than NIS4^{®}. This provides a new valuable tool for diagnosing at-risk NASH subjects.

## Claims

1. A method for the diagnosis, screening, or monitoring of at-risk nonalcoholic steatohepatitis (NASH) in a subject, said method comprising:
- quantifying the levels of miR-34a-5p and YKL-40 in a biological fluid sample of said subject;
- obtaining the gender of said subject;
- combining the quantified levels and the gender in a mathematical function to assign a score;
wherein the score is calculated as $S = \frac{1}{1 + exp \left(-y\right)}$ 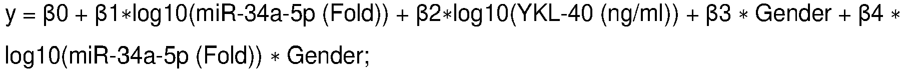 y = β0 ± β1*log10(miR-34a-5p (Fold)) ± β2*log10(YKL-40 (ng/ml)) ± β3 * Gender ± β4 * log10(miR-34a-5p (Fold)) * Gender;
wherein β0 is comprised between -3 and 3;
β1 is comprised between 1 and 5;
β2 is comprised between 0 and 4.5;
β3 is comprised between -2 and 2;
β4 is comprised between -1 and 2; and
wherein Gender is 0 if the subject is a female, or 1 if the subject is a male,
and
- comparing said score with a low cutoff and a high cutoff to determine whether said subject is an at-risk NASH subject,
Wherein the score lower than the low cutoff is indicative a subject not having at-risk NASH, and the score greater or equal to the high cutoff value is indicative of a subject having at-risk NASH, and wherein the low cutoff is comprised between 0.24 and 0.5 and the high cutoff is comprised between 0.6 and 0.95.

2. The method according to claim 1, wherein the biological fluid sample is a blood, serum or plasma sample.

3. The method according to claim 1 or 2, wherein the biological fluid sample is a serum sample.

4. The method according to any one of claims 1 to 3, wherein the subject suffers from obesity, insulin resistance, glucose intolerance, T2DM, prediabetes, dyslipidaemia or hypertriglyceridaemia.

5. A computer program comprising instructions that, when executed by a processor/processing means, cause the processor/processing means to:
- receive quantified levels of miR-34a-5p and YKL-40;
- receive the gender of the subject;
- calculate a score from these quantified levels and gender of the subject, from a mathematical function wherein the score is calculated as $S = \frac{1}{1 + exp \left(-y\right)}$ 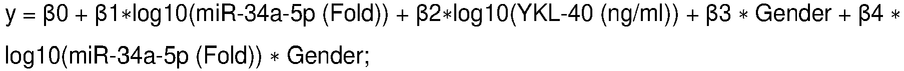 y = β0 ± β1*log10(miR-34a-5p (Fold)) ± β2*log10(YKL-40 (ng/ml)) ± β3 * Gender ± β4 * log10(miR-34a-5p (Fold)) * Gender;
wherein β0 is comprised between -3 and 3;
β1 is comprised between 1 and 5;
β2 is comprised between 0 and 4.5;
β3 is comprised between -2 and 2;
β4 is comprised between -1 and 2; and
wherein Gender is 0 if the subject is a female, or 1 if the subject is a male; and
- assign the subject into the group of at-risk subjects or not at-risk subjects based upon the calculated score compared to a predetermined low cutoff value and a predetermined high cutoff value,
wherein the score lower than the low cutoff is indicative a subject not having at-risk NASH, and the score greater or equal to the high cutoff value is indicative of a subject having at-risk NASH, wherein the low cutoff is comprised between 0.24 and 0.5 and the high cutoff is comprised between 0.6 and 0.95.

6. A computer readable medium comprising the computer program according to claim 5.

7. The computer readable medium according to claim 6, which is a non-transitory medium or a storage medium.

8. An anti-NASH or anti-fibrotic agent for use in the treatment of at-risk NASH in a subject in need thereof,
wherein the anti-NASH agent is selected from pegbelfermin, cenicriviroc, dapagliflozin, dulaglutide, empagliflozin, fenofibrate, lanifibranor, liraglutide, obeticholic acid, pioglitazone, resmetirom, saroglitazar magnesium, seladelpar, semaglutide, sitagliptin, TERN-101, TERN-201 and tropifexor, and
wherein the subject has been classified as having at-risk NASH thanks to the method according to any one of claims 1 to 4.

## Patentansprüche

1. Verfahren zur Diagnose, zum Screening oder zur Überwachung eines Risikos für nichtalkoholische Steatohepatitis (NASH) bei einer Testperson, wobei das Verfahren Folgendes umfasst:
- Quantifizierung der miR-34a-5p- und YKL-40-Konzentrationen in einer biologischen Flüssigkeitsprobe der Testperson;
- Bestimmung des Geschlechts dieser Testperson;
- Kombination der quantifizierten Konzentrationen und des Geschlechts in einer mathematischen Funktion zur Berechnung eines Scores; wobei der Score wie folgt berechnet wird: $S = \frac{1}{1 + exp \left(-y\right)}$ 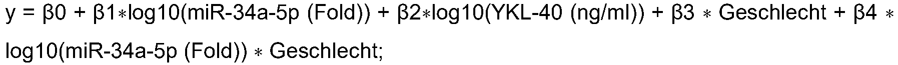 y = β0 + β1∗log10(miR-34a-5p (Fold)) + β2∗log10(YKL-40 (ng/ml)) + β3 ∗ Geschlecht + β4 ∗ log10(miR-34a-5p (Fold)) ∗ Geschlecht;
wobei β0 zwischen -3 und 3 liegt;
β1 zwischen 1 und 5 liegt;
β2 zwischen 0 und 4,5 liegt;
β3 zwischen -2 und 2 liegt;
β4 zwischen -1 und 2 liegt; und
wobei Geschlecht 0 ist, wenn die Testperson weiblich ist, und 1, wenn die Testperson männlich ist,
und
- wobei dieser Wert mit einem unteren und einem oberen Grenzwert verglichen wird, um festzustellen, ob diese Testperson einem NASH-Risiko ausgesetzt ist,
wobei ein Wert unterhalb des unteren Grenzwerts darauf hinweist, dass die Testperson keinem NASH-Risiko ausgesetzt ist, und ein Wert oberhalb oder gleich dem oberen Grenzwert darauf hinweist, dass die Testperson einem NASH-Risiko ausgesetzt ist, und wobei der untere Grenzwert zwischen 0,24 und 0,5 und der obere Grenzwert zwischen 0,6 und 0,95 liegt.

2. Das Verfahren nach Anspruch 1, wobei es sich bei der biologischen Flüssigkeitsprobe um eine Blut-, Serum- oder Plasmaprobe handelt.

3. Das Verfahren nach Anspruch 1 oder 2, wobei es sich bei der biologischen Flüssigkeitsprobe um eine Serumprobe handelt.

4. Das Verfahren nach einem der Ansprüche 1 bis 3, wobei die Testperson an Adipositas, Insulinresistenz, Glukoseintoleranz, Typ-2-Diabetes, Prädiabetes, Dyslipidämie oder Hypertriglyceridämie leidet.

5. Computerprogramm mit Anweisungen, die, wenn sie von einem Prozessor/einer Verarbeitungseinheit ausgeführt werden, diesen/diese veranlassen:
- quantifizierte Werte von miR-34a-5p und YKL-40 zu empfangen;
- das Geschlecht der Testperson zu empfangen;
- anhand dieser quantifizierten Werte und des Geschlechts der Testperson einen Score mithilfe einer mathematischen Funktion zu berechnen, wobei der Score wie folgt berechnet wird: $S = \frac{1}{1 + exp \left(-y\right)}$ 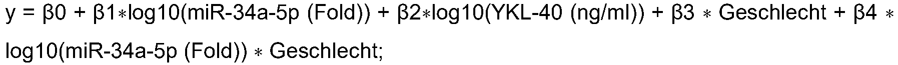 y = β0 + β1∗log10(miR-34a-5p (Fold)) + β2∗log10(YKL-40 (ng/ml)) + β3 ∗ Geschlecht + β4 ∗ log10(miR-34a-5p (Fold)) ∗ Geschlecht;
wobei β0 zwischen -3 und 3 liegt;
β1 zwischen 1 und 5 liegt;
β2 zwischen 0 und 4,5 liegt;
β3 zwischen -2 und 2 liegt;
β4 zwischen -1 und 2 liegt; und
wobei Geschlecht 0 ist, wenn die Testperson weiblich ist, oder 1, wenn die Testperson männlich ist; und
- die Testperson zur Risikogruppe der Testpersonen oder Nicht-Risikogruppe der Testpersonen auf Grundlage des berechneten Wertes im Vergleich zu einem vorab festgelegten unteren Grenzwert und einem vorab festgelegten oberen Grenzwert zuzuordnen, wobei ein Wert unterhalb des unteren Grenzwerts darauf hinweist, dass die Testperson einem NASH-Risiko ausgesetzt ist, und ein Wert oberhalb oder gleich dem oberen Grenzwert darauf hinweist, dass die Testperson einem NASH-Risiko ausgesetzt ist, wobei der untere Grenzwert zwischen 0,24 und 0,5 und der obere Grenzwert zwischen 0,6 und 0,95 liegt.

6. Computerlesbares Medium, das das Computerprogramm nach Anspruch 5 umfasst.

7. Das computerlesbare Medium nach Anspruch 6, das ein nichttransitorisches Medium oder ein Speichermedium ist.

8. Anti-NASH- oder antifibrotisches Mittel zur Verwendung bei der Behandlung von NASH-Risikopatienten,
wobei das Anti-NASH-Mittel ausgewählt ist aus Pegbelfermin, Cenicriviroc, Dapagliflozin, Dulaglutid, Empagliflozin, Fenofibrat, Lanifibranor, Liraglutid, Obeticholsäure, Pioglitazon, Resmetirom, Saroglitazar-Magnesium, Seladelpar, Semaglutid, Sitagliptin, TERN-101, TERN-201 und Tropifexor, und
wobei die Testperson dank des Verfahrens nach einem der Ansprüche 1 bis 4 als einem NASH-Risiko ausgesetzt eingestuft wurde.

## Revendications

1. Méthode pour diagnostiquer, dépister ou surveiller une stéatohépatite non alcoolique (NASH) à risque chez un sujet, ladite méthode comprenant :
- la quantification des niveaux de miR-34a-5p et de YKL-40 dans un échantillon de fluide biologique dudit sujet ;
- l'obtention du sexe dudit sujet ;
- la combinaison des niveaux quantifiés et du sexe dans une fonction mathématique pour attribuer un score ; dans laquelle le score est calculé selon $S = \frac{1}{1 + exp \left(-y\right)}$ 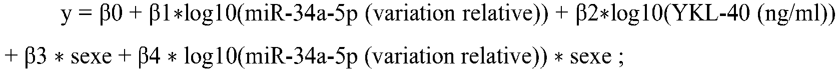 y = β0 + β1∗log10(miR-34a-5p (variation relative)) + β2∗log10(YKL-40 (ng/ml)) + β3 ∗ sexe + β4 ∗ log10(miR-34a-5p (variation relative)) ∗ sexe ;
dans laquelle β0 est compris entre -3 et 3 ;
β1 est compris entre 1 et 5 ;
β2 est compris entre 0 et 4,5 ;
β3 est compris entre -2 et 2 ;
β4 est compris entre -1 et 2 ; et
dans laquelle le sexe vaut 0 si le sujet est de sexe féminin, ou de 1 si le sujet est de sexe masculin,
et
- la comparaison dudit score avec une limite inférieure et une limite supérieure pour déterminer si ledit sujet est un sujet souffrant de NASH à risque,
dans laquelle un score inférieur à la limite inférieure indique qu'un sujet ne souffre pas de NASH à risque, et un score supérieur ou égal à la valeur limite supérieure indique qu'un sujet souffre de NASH à risque, et dans laquelle la limite inférieure est comprise entre 0,24 et 0,5 et la limite supérieure est comprise entre 0,6 et 0,95.

2. Méthode selon la revendication 1, dans laquelle l'échantillon de fluide biologique est un échantillon de sang, de sérum ou de plasma.

3. Méthode selon la revendication 1 ou 2, dans laquelle l'échantillon de fluide biologique est un échantillon de sérum.

4. Méthode selon l'une quelconque des revendications 1 à 3, dans laquelle le sujet souffre d'obésité, de résistance à l'insuline, d'intolérance au glucose, de T2DM (diabète de type 2), de prédiabète, de dyslipidémie ou d'hypertriglycéridémie.

5. Programme informatique comprenant des instructions qui, lorsqu'elles sont exécutées par un processeur/moyen de traitement, amènent le processeur/moyen de traitement à :
- recevoir des niveaux quantifiés de miR-34a-5p et YKL-40 ;
- recevoir le sexe du sujet ;
- calculer un score à partir de ces niveaux quantifiés et du sexe du sujet, à partir d'une fonction mathématique dans laquelle le score est calculé selon $S = \frac{1}{1 + exp \left(-y\right)}$ 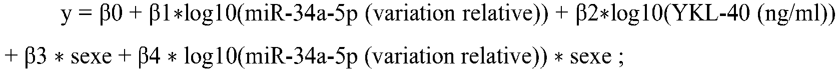 y = β0 + β1∗log10(miR-34a-5p (variation relative)) + β2∗log10(YKL-40 (ng/ml)) + β3 ∗ sexe + β4 ∗ log10(miR-34a-5p (variation relative)) ∗ sexe ;
dans laquelle β0 est compris entre -3 et 3 ;
β1 est compris entre 1 et 5 ;
β2 est compris entre 0 et 4,5 ;
β3 est compris entre -2 et 2 ;
β4 est compris entre -1 et 2 ; et
dans laquelle le sexe vaut 0 si le sujet est de sexe féminin, ou de 1 si le sujet est de sexe masculin ; et
- attribuer le sujet à un groupe de sujets à risque ou un groupe de sujets non à risque sur la base du score calculé comparé à une valeur limite inférieure prédéterminée et à une valeur limite supérieure prédéterminée,
dans lequel un score inférieur à la limite inférieure indique qu'un sujet ne souffre pas de NASH à risque, et un score supérieur ou égal à la valeur limite supérieure indique qu'un sujet souffre de NASH à risque, et dans lequel la limite inférieure est comprise entre 0,24 et 0,5 et la limite supérieure est comprise entre 0,6 et 0,95.

6. Support lisible par ordinateur comprenant le programme informatique selon la revendication 5.

7. Support lisible par ordinateur selon la revendication 6, qui est un support non transitoire ou un support de stockage.

8. Agent anti-NASH ou antifibrotique pour une utilisation dans le traitement d'une NASH à risque chez un sujet en ayant besoin,
dans lequel l'agent anti-NASH est choisi parmi les pegbelfermine, cénicriviroc, dapagliflozine, dulaglutide, empagliflozine, fénofibrate, lanifibranor, liraglutide, acide obéticholique, pioglitazone, resmétirom, saroglitazar magnésium, séladelpar, sémaglutide, sitagliptine, TERN-101, TERN-201 et tropifexor, et
dans lequel le sujet a été classé comme souffrant d'une NASH à risque grâce à la méthode selon l'une quelconque des revendications 1 à 4.
